# EUROPEAN PATENT SPECIFICATION

(11) **EP 4 554 451 B1**
(45) Date of publication and mention of the grant of the patent: **22.04.2026**
(21) Application number: 22751260.5
(22) Date of filing: 13.07.2022
(51) Int. Cl.: A61B 5/00, A61B 5/024, A61B 5/18

(54) **METHOD FOR DRIVER MONITORING SYSTEM AND DRIVER MONITORING SYSTEM**
VERFAHREN FÜR FAHRERÜBERWACHUNGSSYSTEM UND FAHRERÜBERWACHUNGSSYSTEM
PROCÉDÉ POUR UN SYSTÈME DE SURVEILLANCE DE CONDUCTEUR ET SYSTÈME DE SURVEILLANCE DE CONDUCTEUR

(43) Date of publication of application: 21.05.2025
(73) Proprietor: Harman International Industries, Incorporated, Stamford, CT 06901 (US)
(72) Inventor: ZHOU, Rengao, Shanghai 200233 (CN)
(74) Representative: Westphal, Mussgnug & Partner, Patentanwälte mbB
(86) International application number: PCT/CN2022/105381
(87) International publication number: WO 2024/011443

(56) References cited:
- NOWARA E. M.: "Camera-based Vital Signs: Towards Driver Monitoring and Face Liveness Verification", THESIS, 20 August 2018 (2018-08-20), pages 1 - 102, XP055956679
- BOUSEFSAF FREDERIC ET AL: "Automatic Selection of Webcam Photoplethysmographic Pixels Based on Lightness Criteria Photoplethysmographic Pixels Selection Based on Lightness Segmentation", JOURNAL OF MEDICAL AND BIOLOGICAL ENGINEERING, 1 January 2017 (2017-01-01), pages 374 - 385, XP055823858, Retrieved from the Internet <URL:https://hal.archives-ouvertes.fr/hal-03147674/document> [retrieved on 20210713], DOI: 10.1007/s40846-017-0229-1ï¿¿
- MAYANK KUMAR ET AL: "DistancePPG: Robust non-contact vital signs monitoring using a camera", BIOMEDICAL OPTICS EXPRESS, vol. 6, no. 5, 6 April 2015 (2015-04-06), United States, pages 1565, XP055285708, ISSN: 2156-7085, DOI: 10.1364/BOE.6.001565

## Description

### TECHINICAL FIELD

The present disclosure relates to a method adapted for a driver monitoring system (DMS) in a vehicle and the DMS using the method, and specifically relates to the method for accelerating a remote-PhotoPlethysmoGraphy(rPPG) algorithm in the DMS by adaptively dealing with a shadow on a driver's face.

### BACKGROUND

The DMS is a popular system in the field of ADAS (Advanced Driver Assistance System). The DMS monitors and assesses a status of the driver while he/she is driving, and warns the driver if needed and eventually applies brakes if necessary. The DMS is designed to detect two types of distractions: Visual Distraction (also known as 'Eyes on Road'), and Cognitive Distraction (referred to as 'Mind on Road'). Apart from a conventional method of analyzing eye gazes and eye blinks, cognitive load and stress could also be derived from an IBI, InterBeat-Interval (or simply speaking, a speed of heart beats). The rPPG process is a great approach of obtaining the IBI, since it doesn't need to contact the human skin.

The rPPG stands for remote-PhotoPlethysmoGraphy, or simply speaking, a remote heart rate estimation. It is a simple optical technique used to detect volumetric changes in blood in a peripheral circulation. The rPPG algorithm may measure the variance of red, green, and blue light reflection changes from the skin, without contacting. The rPPG algorithm measures a contrast between a specular reflection and a diffused reflection. The specular reflection is a pure light reflection from the skin, while the diffused reflection is a reflection that remains after absorption and scattering by skin tissue, which varies as blood volume changes.

Nowara E. M.: "Camera-based Vital Signs: Towards Driver Monitoring and Face Liveness Verification", Thesis, Rice University, Houston, Texas, 20 August 2018 (2018-08-20), pages 1-102, XP055956679, discloses hardware and software solutions to improve camera-based vital signs monitoring in a car comprising processing of rPPG signals with sliding time windows. For each time window of length T, N rPPG signals, from each facial region, are stacked into a T x N rPPG matrix P. If the signal-to-noise-ratio (SNR) of a region is below a threshold and if the regions maximum amplitude is above a threshold for a given time window the regions are removed before further processing.

However, applying the rPPG algorithm under scenarios of DMS may encounter some problems, among which the most noticeable problem is an insufficiency of computing power of the DMS. Therefore, it is necessary to provide an improved technology.

### SUMMARY

According to one aspect of the disclosure, a method adapted for a driver monitoring system is provided. The method may comprise obtaining sequences of frames, wherein each sequence of frames consists of a plurality of color scalars associated with a plurality of face zones of a driver in a vehicle. For each sequence of frames, the method may determine whether a monitoring function is activated for a first set of face zones that are face zones in shadow; and may perform subsequent steps in response to determining the monitoring function being activated. In some embodiments, the subsequent steps may comprise monitoring the first set of face zones; adaptively determining a second set of face zones that are face zones not in shadow, based on results of the monitoring; and performing calculations for recovering heart beat signals only for the second set of face zones.

According to another aspect of the present disclosure, a driver monitoring system is provided. The driver monitoring system may comprise a camera and a processor. The camera may capture face images of the driver in a vehicle. The processor may be coupled to the camera and obtain sequences of frames from the face images, wherein each sequence of frames consists of a plurality of color scalars associated with a plurality of face zones of the driver. The processor may perform the following for each sequence of frames. The processor may determine whether a monitoring function is activated for a first set of face zones that are face zones in shadow; and may perform subsequent processes in response to determining the monitoring function being activated. In some embodiment, the processor may monitor the first set of face zones; adaptively determine a second set of face zones that are face zones not in shadow, based on results of the monitoring; and perform calculations for recovering heart beat signals only for the second set of face zones.

According to yet another aspect of the present disclosure, a non-transitory computer-readable storage medium comprising computer-executable instructions is provided which, when executed by a computer, causes the computer to perform the method disclosed herein.

### BRIEF DESCRIPTION OF THE DRAWINGS

FIG.1 illustrates an example of a definition of landmarks and generated face zones.
FIG. 2 illustrates an example of the DMS in a vehicle according to one or more embodiments of the present disclosure.
FIG. 3 illustrates an example of the method for accelerating the rPPG process according to one or more embodiments of the present disclosure.
FIG. 4 illustrates another example of the method for accelerating the rPPG process according to one or more embodiments of the present disclosure.

To facilitate understanding, identical reference numerals have been used, where possible, to designate identical elements that are common to the figures. It is contemplated that elements disclosed in one embodiment may be beneficially utilized on other embodiments without specific recitation. The drawings referred to here should not be understood as being drawn to scale unless specifically noted. Also, the drawings are often simplified and details or components omitted for clarity of presentation and explanation. The drawings and discussion serve to explain principles discussed below, where like designations denote like elements.

### DETAILED DESCRIPTION OF THE PREFERRED EMBODIMENTS

Examples will be provided below for illustration. The descriptions of the various examples will be presented for purposes of illustration, but are not intended to be exhaustive or limited to the embodiments disclosed. Many modifications and variations will be apparent to those of ordinary skill in the art without departing from the scope of the described embodiments.

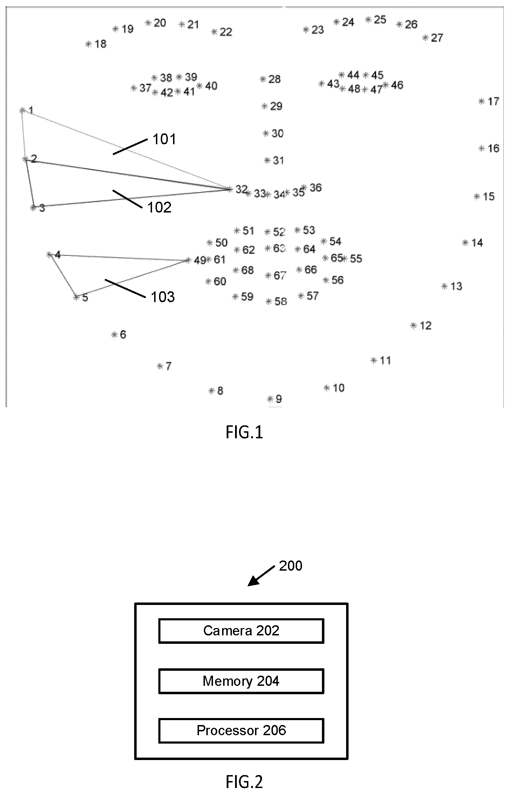

In the present disclosure, a novel method and system is provided to accelerate the rPPG algorithm to address issues faced when porting the algorithm into the DMS of a vehicle. This is achieved by adaptively dealing with a shadow on the driver's face. Particularly, face zones in shadow may be determined and monitored continuously so that only data associated with face zones not in shadow will be used to perform the rPPG algorithm (e.g., perform calculations or processes for recovering heart beat signals), instead of all data associated with all the face zones of the driver's face. In other word, data associated with face zones in shadow will be excluded from calculations for recovering heart beat signals. The method and system in the present disclosure may further dynamically determine and adjust a set of face zones not in shadow by monitoring the face zones in shadow. If the shadow or a part of the shadow disappear, the corresponding face zones which were considered as face zones in shadow will be reconsidered as face zones not in shadow and will be put back to the calculations for recovering heart beat signals. The method and system proposed in the present disclosure may adaptively implement that only the data associated with the face zones not in shadow are calculated, thereby saving calculation time, resources and power consumption. The method and system will be explained in details referring to FIGS. 1-4 as follows.

Under the scenarios of DMS, the rPPG process takes color scalars of different predefined face zones as inputs, wherein the color scalars are values related to the light reflection changes from the skin. These predefined face zones are generated by linking detected face landmarks. FIG.1 illustrates an example of the definition of landmarks and the generated face zones. As shown in the example of FIG.1, for a face, 68 face landmarks may be detected, and accordingly the face may be partitioned into 51 predefined face zones. In FIG. 1, only three face zones are marked with triangles for clarity of presentation, for example, identified by reference numerals 101, 102 and 103.

Taking the generated face zones shown in FIG. 1 as an example, in the rPPG process, a sequence of frames may be considered or processed together, for color scalars of every 51 predefined face zones. Thus, there will be a total of 51 arrays of color scalars at a time. Optionally, for those face zones in which the color scalars are bad (usually because of failed detection, or because of leafy shades on the driver's face, and so on), an interpolation will be used to make up for the bad ones. After that, a proposed method with some calculations will be further performed for recovering heart beat signals, which will be described in details below.

FIG. 2 illustrates an exemplary diagram of the DMS in a vehicle according to one or more embodiments of the present disclosure. As shown in FIG.2, the DMS 200 may include a camera 202, which may capture face images of an occupant, such as a driver or a passenger in the vehicle. The camera 202 may be positioned in locations in the vehicle that may monitor the face of the occupant. The camera 202 may include one or more optical (e.g., visible light) cameras, one or more infrared (IR) cameras, or a combination of optical and IR cameras having one or more view angles. In some examples, the camera 202 may include one or more lenses and one or more image sensors. In some examples, the camera 202 may be a digital camera configured to acquire a series of images (e.g., frames) at a programmable frequency (e.g., frame rate). In some examples, the frame rate may be selected based on a processing speed of a processor 206 included in the DMS 200.

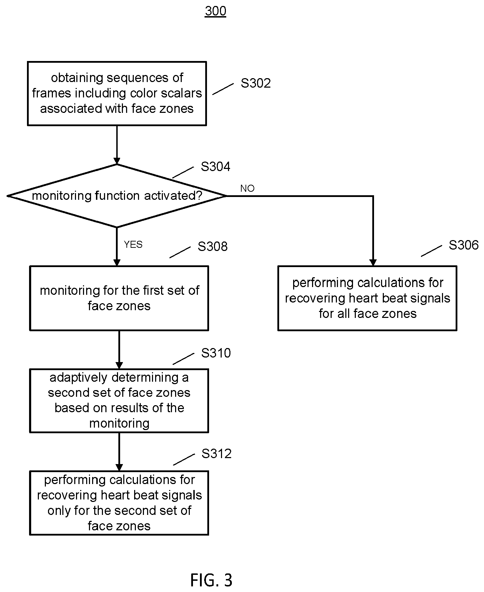

The processor 206 may be configured to execute machine readable instructions stored in a memory 204. The processor 206 may be electronically and/or communicatively coupled to the camera 202, and may process and analyze face images received from the camera to recover the heart beat signals. In some examples, the processor 206 may obtain and process sequences of frames from the face images, wherein each sequence of frames consists of N arrays of color scalars corresponding to N face zones of the driver. N is an integer greater than one and represents an amount of face zones of the occupant. The generation of face zones may be similar to, or the same as, the example described in reference with FIG. 1. The amount of N may be preset. The processor 206 may be configured to perform the methods, as will be elaborated hereafter with respect to FIGS. 3-4.

The processor 206 may be single core or multi-core, and the programs executed by processor 206 may be configured for parallel or distributed processing. The processor 206 may be any technically feasible hardware unit configured to carry out processing functions and execute software applications, including without limitation, a central processing unit (CPU), a microcontroller unit (MCU), an application specific integrated circuit (ASIC), a digital signal processor (DSP) chip, a field-programmable gate array (FPGA), a graphic board, and so forth.

The memory 204 may include any non-transitory tangible computer readable medium in which programming instructions are stored. As used herein, the term "tangible computer readable medium" is expressly defined to include any type of computer readable storage. The example methods described herein may be implemented using coded instruction (e.g., computer readable instructions) stored on a non-transitory computer readable medium such as a flash memory, a read-only memory (ROM), a random-access memory (RAM), a cache, or any other storage media in which information is stored for any duration (e.g. for extended period time periods, permanently, brief instances, for temporarily buffering, and/or for caching of the information). Computer memory of computer readable storage mediums as referenced herein may include volatile and non-volatile or removable and non-removable media for a storage of electronically formatted information, such as computer readable program instructions or modules of computer readable program instructions, data, etc. that may be stand-alone or as part of a computing device. Examples of computer memory may include any other medium which can be used to store the desired electronic format of information and which can be accessed by the processor or processors or at least a portion of a computing device.

FIG. 3 illustrates an example of the method 300 for accelerating the rPPG process according to one or more embodiments of the present disclosure. The method 300 will be described with respect to the system of FIG.2. For example, the method 300 may be executed by the processor 206 based on instructions/codes stored on the memory 204 of the DMS 200. However, it may be understood that the method may be implemented with other systems and components without departing from the scope of the present disclosure.

At S302, the processor 206 may obtain sequences of frames from the face images. Each sequence of frames may include a plurality of color scalars associated with a plurality of face zones of the driver. For example, assuming there are 51 face zones, there will be a total of 51 arrays of color scalars at a time, wherein one array corresoonds to one face zone. It can be understood that for example, assuming that the length of frame sequence is 512, then each array includes 512 color scalars and one array of 512 color scalars corresponds to one face zone.

At S304, the processor 206 may determine, for each sequence of frames, whether a monitoring function is activated for some face zones in shadow. If the determination is "NO", which means no shadow exists, the method then proceeds to S306.

At S306, the processor 206 may use all the color scalars associated with all the face zones to perform calculations or processes for recovering heart beat signals. In some examples, FFT (Fast Fourier Transfer) calculations may be applied over the sequence of frames including color scalars corresponding to all the face zones. Based on the results of FFT calculations, SNR (Signal-Noise Ratio) values for all the face zones may be calculated. Each FFT result may also be fed into a Neural Network to derive each quality value for each face zone. Thus, quality values for all the face zones may be obtained. Based on the color scalars, the calculated SNR values and the calculated quality values, zone values may be calculated. Taking the 51 face zones as an example, 51 zone values for 51 face zones will be obtained. For example, each zone value may be calculated by ColorScalar*SNR*Quality, wherein ColorScalar represents the value of the color scalar, SNR represents the calculated SNR value, Quality represents the calculated quality value, and the symbol * represents for a multiplication operator. Then, the calculated zone values for all the face zones may be added up to obtain the final values indicating heart beat signals. Based on the obtained final values, the heart beat signals may be recovered.

Returning to S304, if the determination is "YES", the method then proceeds to S308. At S308, the processor 206 may monitor the face zones in shadow. In some embodiments, the monitoring for the face zones in shadow may comprise continuously monitoring these face zones to determine whether some changes for these face zones occur, for example, one or more face zones are no longer located in the shadow. In other words, it may be determined whether one or more of these monitored face zones should be reconsidered as face zones not in shadow. In some embodiments, the monitoring for the face zones in shadow may comprise calculating SNR values for these monitored face zones in shadow

The method then proceeds to S310. At S310, a set of face zones not in shadow may be determined or adjusted adaptively according to the results of the monitoring. In some embodiments, the processor 206 may compare each of the calculated SNR values with a SNR threshold. If none of the calculated SNR values is larger than the SNR threshold, which means these face zones are still in the shadow, then the set of face zones not in shadow will be maintained. If one or more of the calculated SNR values are larger than or equal to the SNR threshold, then the set of face zones not in shadow will be adjusted. In some examples, the processor 206 may determine to which face zone or zones the calculated SNR or SNRs larger than or equal to the threshold correspond, and then put the determined one or more face zones into the set of face zones not in shadow. It can be understood that the set of face zones not in shadow and the set of face zones in shadow constitute the entire face zones.

The method then proceeds to S312. At S312, the processor may perform subsequent calculations for recovering heart beat signals only for the set of face zones not in shadow. In some embodiments, the processor 206 may only calculate SNR values for the set of face zones not in shadow and quality values for the set of face zones not in shadow, based on the FFT results as described above. The processor 206 may further calculate zone values for the set of face zones not in shadow, based on the color scalars associated with the face zones not in shadow, the calculated SNR values and the calculated quality values. Further, the processor 206 may add up the calculated zone values to obtain final values, and recover the heart beat signals based on the obtained final values. In some examples, the processor 206 may further determine whether there is one or more zone values smaller than a predefined threshold. If there is one or more zone values smaller than a predefined threshold, the monitoring function for those face zones associated with the one or more zone values smaller than the threshold will be activated for the following sequences of frames.

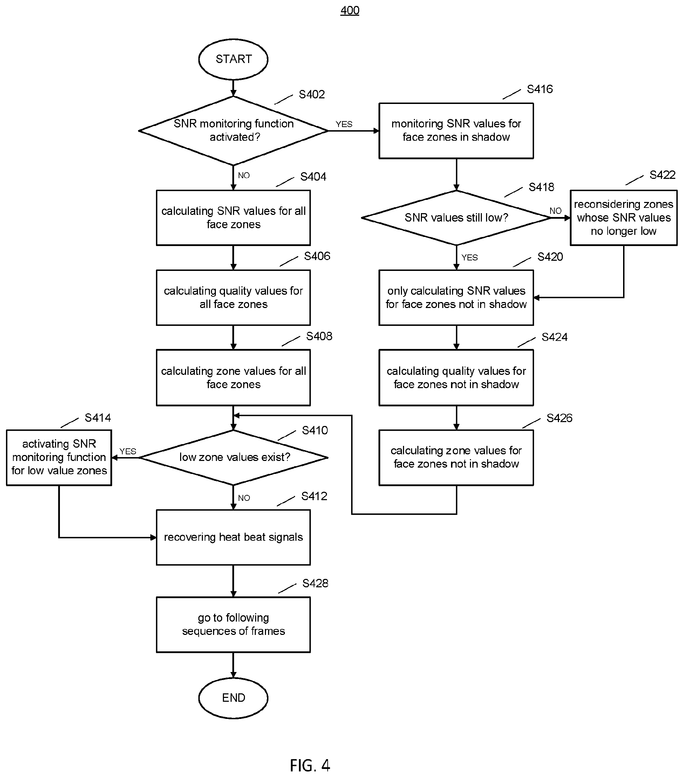

FIG. 4 illustrates another example method 400 for accelerating the rPPG process according to one or more embodiments of the present disclosure. The method 400 may be performed for each sequence of frames obtained from the face images. As described with respect to FIG.3, each sequence of frames may include a plurality of color scalars associated with a plurality of face zones of the driver.

At S402, a determination may be first performed to adjudge whether the monitoring function is activated for some face zones in shadow. If the determination is "NO", which means no shadow exists, the method then proceeds to S404.

At S404, the SNR values for all face zones may be calculated, and at S406, the quality values for all the face zones may be obtained. It is contemplated that the steps of S404 and S406 shown in succession may, in fact, be executed substantially concurrently, or the steps may sometimes be executed in the reverse order. At S408, zone values for all face zones will be obtained based on the color scalars, the calculated SNR values and the calculated quality values. The calculation details of S404~S408 may be similar to, or the same as, the descriptions with respect to S306 of FIG.3. The method then proceeds to S410.

At S410, the method determines whether one or more low zone values exist. Ideally, the light conditions of all face zones for the sequence of frames (e.g., of every individual 51 face zones) would be expected to be similar, resulting the SNR values and signal quality values of each of 51 face zones being close to each other. Consequently, the 51 zone values each of which is calculated by (ColorScalar*SNR*Quality), would also be close to each other among all the 51 face zones. However, in real driving conditions, shadows might exist on the driver's face, for example, caused by the vehicle's sun visor or by trees on avenue. Unlike tree shadows that move quickly, shadows caused by the sun visor usually persist on specific locations on the driver's face. As a result, these specific locations in shadow (for example, on one or more face zones in shadow) would have one or more low values of SNR and Quality, and thus one or more low zone values, for a long time within a duration of the entire frame sequence (i.e., the length of the frame sequence). Under this scenario, when executing the step of calculating final values, these low zone values would be considered as low-quality 'noise'. Thus, the computation power (for calculating SNR, Quality) for these face zones in shadow would waste in vain. In order to solve this problem, the method of the present disclosure determines, for example at S410, whether one or more low zone values exist. Since the sun visor's shadow would usually persist on specific face zones (most likely on the upper part of the face), and the shaded area would not change frequently, it is rational to exclude the shaded areas out of the calculating for the following frames afterwards.

Returning to S410, a criteria for 'low' here may be various. In some examples, the criteria may be a comparison to a predefined threshold. If the one or more calculated zone values are smaller than the predefined threshold, then they may be determined to be 'low' zone values. In some examples, the criteria may be a comparison to a proportional threshold that depends on a ratio of one zone value to other valid zone values. For example, if one or more zone values are less than 1/10 of other valid zone values, then the one or more zone values may be determined to be 'low' zone values.

If it determines at S410 that there is not any low zone value, the method then proceeds to S412. At S412, the method may recover heart beat signals. In some examples, at S412, the method may comprise adding up the zone values calculated at S408 to obtain final values for all the face zones, and recovering the heart beat signals based on the obtained final values. Then, the method proceeds to S428 to go to the following sequences of frames.

If it determines at S410 that there is one or more low zone values, the method then proceeds to S414. At S414, the method may activate the SNR monitoring function for these face zones with low zone values. Then, the method proceeds to S412, and then to S428.

Returning to S402, if the determination result of S402 is "YES", the method then proceeds to S416. At S416, the method monitors SNR values only for the face zones in shadow which were identified in the process for the previous sequence(s) of frames. That means, in the SNR monitoring function, only the SNR values of those shadow-like areas that were identified may be calculated. In some examples, the SNR monitoring function (i.e., the calculation of SNR values for the one or more face zones in shadow) may be executed at a lower frequency than the frame rate. In some examples, for all the identified face zones in shadow, SNR values may be calculated. In some examples, the method may randomly select several face zones from the identified face zones in shadow to calculate their SNR values, instead of calculating the SNR values for every face zones in shadow.

At S418, the method further determines whether the calculated SNR values at S416 are still 'low'. A criteria for 'low' here may be various. In some examples, the criteria may be a comparison to a predefined SNR threshold. If the one or more calculated SNR values are smaller than the predefined SNR threshold, then they may be determined to be still 'low'. In some examples, the criteria may be a comparison to a proportional threshold that depends on a ratio of one SNR value to other valid SNR values. For example, if one or more SNR values are less than 1/10 of other valid SNR values, then the one or more SNR values may be determined to be still 'low'.

If the method determines at S418 that all SNR values are still low, then the method proceeds to S420. If the method determines at S418 that one or more SNR values are no longer low, then the method proceeds to S422. At S422, the face zones corresponding to the one or more SNR values that are no longer low may be reconsidered as the face zones that are not in shadow. Then, the method proceeds to S420.

At S420, only the SNR values of face zones not in shadow may be calculated. Then, the method proceeds to S424. At S424, quality values for the face zones not in shadow may be obtained. Then, the method proceeds to S426. At S426, zone values for the face zones not in shadow may be calculated based on the color scalars only associated with the face zones not in shadow, the SNR values calculated at S420 and the quality values calculated at S424. Then, the method proceeds to S410 to determine whether one or more low zone values exist among the face zones.

If it determines at S410 that there is not any low zone value, the method then proceeds to S412. At S412, the method may recover heart beats. In some examples, the method at S412 may comprise adding up the zone values calculated at S426 to obtain final values for the face zones not in shadow, and recovering the heart beat signals based on the obtained final values. Then, the method proceeds to S428 to go to the following sequences of frames.

If it determines that there is one or more low zone value at S410, the method then proceeds to S414. At S414, the method may activate the SNR monitoring function for those face zones with low zone values. The method proceeds to S412, and then to S428 to perform processing of the subsequent sequence of frames.

The descriptions of the various embodiments have been presented for purposes of illustration, but are not intended to be exhaustive or limited to the embodiments disclosed. The terminology used herein was chosen to best explain the principles of the embodiments, the practical application or technical improvement over technologies found in the marketplace, or to enable others of ordinary skill in the art to understand the embodiments disclosed herein.

In the preceding, reference is made to embodiments presented in this disclosure. However, the scope of the present disclosure is not limited to specific described embodiments. Instead, any combination of the preceding features and elements, whether related to different embodiments or not, is contemplated to implement and practice contemplated embodiments. Furthermore, although embodiments disclosed herein may achieve advantages over other possible solutions or over the prior art, whether or not a particular advantage is achieved by a given embodiment is not limiting of the scope of the present disclosure. Thus, the preceding aspects, features, embodiments and advantages are merely illustrative and are not considered elements or limitations of the appended claims except where explicitly recited in a claim(s).

Aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module", "unit" or "system."

The present disclosure may be a system, a method, and/or a computer program product. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals *per se,* such as radio waves or other freely propagating electromagnetic waves, electromagnetic waves propagating through a waveguide or other transmission media (*e.g.*, light pulses passing through a fiber-optic cable), or electrical signals transmitted through a wire.

Computer readable program instructions described herein can be downloaded to respective calculating/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

The flowchart and block diagrams in the drawings illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

While the foregoing is directed to embodiments of the present disclosure, other and further embodiments of the disclosure may be devised without departing from the basic scope thereof, and the scope thereof is determined by the claims that follow.

## Claims

1. A method for a driver monitoring system, comprising:
obtaining sequences of frames, each sequence of frame including a plurality of color scalars associated with a plurality of face zones of a driver in a vehicle;
determining, for each sequence of frames, whether a monitoring function is activated for a first set of face zones that are face zones in shadow; and
in response to determining the monitoring function being activated, performing the following:
monitoring the first set of face zones;
adaptively determining a second set of face zones that are face zones not in shadow, based on results of the monitoring; and
performing calculations for recovering heart beat signals only for the second set of face zones.

2. The method according to claim 1, wherein the monitoring the first set of face zones comprises calculating first SNR values for the first set of face zones.

3. The method according to claim 2, wherein the adaptively determining the second set of face zones based on results of the monitoring comprises:
comparing each first SNR value with an SNR threshold;
maintaining the second set of face zones in response to all first SNR values being less than the SNR threshold; and
adjusting the second set of face zones in response to one or more first SNR values being greater than or equal to the SNR threshold.

4. The method according to claim 3, wherein the adjusting the second set of face zones comprises:
determining one or more face zones associated with the one or more first SNR values greater than or equal to the SNR threshold; and
putting the determined one or more face zones into the second set of face zones.

5. The method according to claim 1, wherein the performing the calculations for recovering the heart beat signals only for the second set of face zones comprises:
calculating zone values for the second set of face zones;
adding up the calculated zone values to obtain final values; and
recovering the heart beat signals based on the obtained final values.

6. The method according to claim 5, wherein the calculating the zone values for the second set of face zones comprises:
calculating second SNR values for the second set of face zones;
calculating second quality values for the second set of face zones; and
calculating the zone values for the second set of face zones, based on the color scalars associated with the second set of face zones, the calculated second SNR values and the calculated second quality values.

7. The method according to claim 5, further comprises:
determining whether there is one or more zone values less than a threshold;
putting one or more face zones associated with the one or more zone values less than the threshold into the first set of face zones; and
activating the monitoring function for the first set of face zones.

8. The method according to claim 1, further comprises performing calculations for recovering heart beat signals for all the face zones in response to determining the monitoring function being not activated.

9. The method according to any one of claims 1-8, further comprises:
capturing face images of the driver by a camera of the driver monitoring system; and
obtaining the sequence of frames from the face images.

10. A driver monitoring system comprising:
a camera configured to capture face images of a driver in a vehicle; and
a processor coupled to the camera and configured to:
obtain sequences of frames from the face images, wherein each sequence of frames includes a plurality of color scalars associated with a plurality of face zones of the driver;
determine, for each sequence of frames, whether a monitoring function is activated for a first set of face zones that are face zones in shadow; and
in response to determining the monitoring function being activated, perform the following:
monitoring the first set of face zones;
adaptively determining a second set of face zones that are face zones not in shadow, based on results of the monitoring; and
performing calculations for recovering heart beat signals only for the second set of face zones.

11. The driver monitoring system according to claim 10, wherein the processor is configured to calculate first SNR values for the first set of face zones.

12. The driver monitoring system according to claim 11, wherein the processor is configured to:
compare each first SNR value with an SNR threshold;
maintain the second set of face zones in response to all first SNR values being less than the SNR threshold; and
adjust the second set of face zones in response to one or more first SNR values being greater than or equal to the SNR threshold.

13. The driver monitoring system according to claim 12, wherein the processor is configured to:
determine one or more face zones associated with the one or more first SNR values greater than or equal to the SNR threshold; and
put the determined one or more face zones into the second set of face zones.

14. The driver monitoring system according to claim 10, wherein the processor is configured to:
calculate zone values for the second set of face zones;
add up the calculated zone values to obtain final values; and
recover the heart beat signals based on the obtained final values.

15. A computer-readable storage medium comprising computer-executable instructions which, when executed by a computer, causes the computer to perform the method according to any one of claims 1-9.

## Patentansprüche

1. Verfahren für ein Fahrerüberwachungssystem, umfassend:
Erhalten von Sequenzen von Frames, wobei jede Framesequenz eine Vielzahl von Farbskalaren einschließt, die mit einer Vielzahl von Gesichtszonen eines Fahrers in einem Fahrzeug verbunden sind;
Bestimmen, ob eine Überwachungsfunktion für einen ersten Satz von Gesichtszonen, die Gesichtszonen in einem Schatten sind, aktiviert ist, für jede Sequenz von Frames; und
als Reaktion auf das Bestimmen, dass die Überwachungsfunktion aktiviert ist, Durchführen des Folgenden:
Überwachen des ersten Satzes von Gesichtszonen;
adaptives Bestimmen eines zweiten Satzes von Gesichtszonen, die Gesichtszonen sind, die nicht in dem Schatten sind, basierend auf Ergebnissen des Überwachens; und
Durchführen von Berechnungen zum Wiederherstellen von Herzschlagsignalen nur für den zweiten Satz von Gesichtszonen.

2. Verfahren nach Anspruch 1, wobei das Überwachen des ersten Satzes von Gesichtszonen ein Berechnen erster SNR-Werte für den ersten Satz von Gesichtszonen umfasst.

3. Verfahren nach Anspruch 2, wobei das adaptive Bestimmen des zweiten Satzes von Gesichtszonen basierend auf Ergebnissen des Überwachens Folgendes umfasst:
Vergleichen jedes ersten SNR-Wertes mit einem SNR-Schwellenwert;
Beibehalten des zweiten Satzes von Gesichtszonen als Reaktion darauf, dass alle ersten SNR-Werte kleiner als der SNR-Schwellenwert sind; und
Anpassen des zweiten Satzes von Gesichtszonen als Reaktion darauf, dass ein oder mehrere erste SNR-Werte größer oder gleich dem SNR-Schwellenwert sind.

4. Verfahren nach Anspruch 3, wobei das Anpassen des zweiten Satzes von Gesichtszonen Folgendes umfasst:
Bestimmen einer oder mehrerer Gesichtszonen, die mit dem einen oder den mehreren ersten SNR-Werten verbunden sind, die größer oder gleich dem SNR-Schwellenwert sind; und
Einfügen der bestimmten einen oder mehreren Gesichtszonen in den zweiten Satz von Gesichtszonen.

5. Verfahren nach Anspruch 1, wobei das Durchführen der Berechnungen zum Wiederherstellen der Herzschlagsignale nur für den zweiten Satz von Gesichtszonen Folgendes umfasst:
Berechnen von Zonenwerten für den zweiten Satz von Gesichtszonen;
Addieren der berechneten Zonenwerte, um Endwerte zu erhalten; und
Wiederherstellen der Herzschlagsignale basierend auf den erhaltenen Endwerten.

6. Verfahren nach Anspruch 5, wobei das Berechnen der Zonenwerte für den zweiten Satz von Gesichtszonen Folgendes umfasst:
Berechnen zweiter SNR-Werte für den zweiten Satz von Gesichtszonen;
Berechnen zweiter Qualitätswerte für den zweiten Satz von Gesichtszonen; und
Berechnen der Zonenwerte für den zweiten Satz von Gesichtszonen basierend auf den mit dem zweiten Satz von Gesichtszonen verbundenen Farbskalaren, den berechneten zweiten SNR-Werten und den berechneten zweiten Qualitätswerten.

7. Verfahren nach Anspruch 5, das ferner Folgendes umfasst:
Bestimmen, ob ein oder mehrere Zonenwerte vorhanden sind, die kleiner als ein Schwellenwert sind;
Einfügen einer oder mehrerer Gesichtszonen, die mit dem einen oder den mehreren Zonenwerten verbunden sind, die kleiner als der Schwellenwert sind, in den ersten Satz von Gesichtszonen; und
Aktivieren der Überwachungsfunktion für den ersten Satz von Gesichtszonen.

8. Verfahren nach Anspruch 1, das ferner ein Durchführen von Berechnungen zum Wiederherstellen von Herzschlagsignalen für all die Gesichtszonen als Reaktion auf das Bestimmen umfasst, dass die Überwachungsfunktion nicht aktiviert ist.

9. Verfahren nach einem der Ansprüche 1 bis 8, das ferner Folgendes umfasst:
Aufnehmen von Gesichtsbildern des Fahrers durch eine Kamera des Fahrerüberwachungssystems; und
Erhalten der Sequenz von Frames aus den Gesichtsbildern.

10. Fahrerüberwachungssystem, umfassend:
eine Kamera, die dazu konfiguriert ist, Gesichtsbilder eines Fahrers in einem Fahrzeug aufzunehmen; und
einen Prozessor, der mit der Kamera verbunden und zu Folgendem konfiguriert ist:
Erhalten von Sequenzen von Frames aus den Gesichtsbildern, wobei jede Sequenz von Frames eine Vielzahl von Farbskalaren einschließt, die mit einer Vielzahl von Gesichtszonen des Fahrers verbunden sind;
Bestimmen, ob eine Überwachungsfunktion für einen ersten Satz von Gesichtszonen, die Gesichtszonen in einem Schatten sind, aktiviert ist, für jede Sequenz von Frames; und
als Reaktion auf das Bestimmen, dass die Überwachungsfunktion aktiviert ist, Durchführen des Folgenden:
Überwachen des ersten Satzes von Gesichtszonen;
adaptives Bestimmen eines zweiten Satzes von Gesichtszonen, die Gesichtszonen sind, die nicht in dem Schatten sind, basierend auf Ergebnissen des Überwachens; und
Durchführen von Berechnungen zum Wiederherstellen von Herzschlagsignalen nur für den zweiten Satz von Gesichtszonen.

11. Fahrerüberwachungssystem nach Anspruch 10, wobei der Prozessor dazu konfiguriert ist, erste SNR-Werte für den ersten Satz von Gesichtszonen zu berechnen.

12. Fahrerüberwachungssystem nach Anspruch 11, wobei der Prozessor zu Folgendem konfiguriert ist:
Vergleichen jedes ersten SNR-Wertes mit einem SNR-Schwellenwert;
Beibehalten des zweiten Satzes von Gesichtszonen als Reaktion darauf, dass alle ersten SNR-Werte kleiner als der SNR-Schwellenwert sind; und
Anpassen des zweiten Satzes von Gesichtszonen als Reaktion darauf, dass ein oder mehrere erste SNR-Werte größer oder gleich dem SNR-Schwellenwert sind.

13. Fahrerüberwachungssystem nach Anspruch 12, wobei der Prozessor zu Folgendem konfiguriert ist:
Bestimmen einer oder mehrerer Gesichtszonen, die mit dem einen oder den mehreren ersten SNR-Werten verbunden sind, die größer oder gleich dem SNR-Schwellenwert sind; und
Einfügen der bestimmten einen oder mehreren Gesichtszonen in den zweiten Satz von Gesichtszonen.

14. Fahrerüberwachungssystem nach Anspruch 10, wobei der Prozessor zu Folgendem konfiguriert ist:
Berechnen von Zonenwerten für den zweiten Satz von Gesichtszonen;
Addieren der berechneten Zonenwerte, um Endwerte zu erhalten; und
Wiederherstellen der Herzschlagsignale basierend auf den erhaltenen Endwerten.

15. Computerlesbares Speichermedium, umfassend computerausführbare Anweisungen, die, wenn sie durch einen Computer ausgeführt werden, den Computer dazu veranlassen, das Verfahren nach einem der Ansprüche 1 bis 9 durchzuführen.

## Revendications

1. Procédé pour un système de surveillance de conducteur, comprenant :
l'obtention de séquences d'images, chaque séquence d'images comportant une pluralité de scalaires de couleur associés à une pluralité de zones de visage d'un conducteur dans un véhicule ;
le fait de déterminer, pour chaque séquence d'images, si une fonction de surveillance est activée pour un premier ensemble de zones de visage qui sont des zones de visage ombragées ; et
en réponse à la détermination que la fonction de surveillance est activée, la réalisation des opérations suivantes :
la surveillance du premier ensemble de zones de visage ;
la détermination adaptative d'un second ensemble de zones de visage qui sont des zones de visage non ombragées, sur la base de résultats de la surveillance ; et
la réalisation de calculs de récupération de signaux de battements cardiaques uniquement pour le second ensemble de zones de visage.

2. Procédé selon la revendication 1, dans lequel la surveillance du premier ensemble de zones de visage comprend le calcul de premières valeurs SNR pour le premier ensemble de zones de visage.

3. Procédé selon la revendication 2, dans lequel la détermination adaptative du second ensemble de zones de visage sur la base de résultats de la surveillance comprend :
la comparaison de chaque première valeur SNR avec un seuil SNR ;
le maintien du second ensemble de zones de visage en réponse au fait que toutes les premières valeurs SNR sont inférieures au seuil de SNR ; et
l'ajustement du second ensemble de zones de visage en réponse à une ou plusieurs premières valeurs SNR supérieures ou égales au seuil SNR.

4. Procédé selon la revendication 3, dans lequel l'ajustement du second ensemble de zones de visage comprend :
la détermination d'une ou de plusieurs zones de visage associées aux une ou plusieurs premières valeurs SNR supérieures ou égales au seuil SNR ; et
le placement des une ou plusieurs zones de visage déterminées dans le second ensemble de zones de visage.

5. Procédé selon la revendication 1, dans lequel la réalisation des calculs de récupération des signaux de battements cardiaques uniquement pour le second ensemble de zones de visage comprend :
le calcul de valeurs de zone pour le second ensemble de zones de visage ;
l'addition des valeurs de zone calculées pour obtenir des valeurs finales ; et
la récupération des signaux de battements cardiaques sur la base des valeurs finales obtenues.

6. Procédé selon la revendication 5, dans lequel le calcul des valeurs de zone pour le second ensemble de zones de visage comprend :
le calcul de secondes valeurs SNR pour le second ensemble de zones de visage ;
le calcul de secondes valeurs de qualité pour le second ensemble de zones de visage ; et
le calcul des valeurs de zone pour le second ensemble de zones de visage, sur la base des scalaires de couleur associés au second ensemble de zones de visage, des secondes valeurs SNR calculées et des secondes valeurs de qualité calculées.

7. Procédé selon la revendication 5, comprenant en outre :
le fait de déterminer s'il existe une ou plusieurs valeurs de zone inférieures à un seuil ;
le placement d'une ou de plusieurs zones de visage associées aux une ou plusieurs valeurs de zone inférieures au seuil dans le premier ensemble de zones de visage ; et
l'activation de la fonction de surveillance pour le premier ensemble de zones de visage.

8. Procédé selon la revendication 1, comprenant en outre la réalisation de calculs de récupération de signaux de battements cardiaques pour toutes les zones de visage en réponse à la détermination que la fonction de surveillance n'est pas activée.

9. Procédé selon l'une quelconque des revendications 1 à 8, comprenant en outre :
la capture d'images de visage du conducteur par une caméra du système de surveillance de conducteur ; et
l'obtention de la séquence d'images à partir des images de visage.

10. Système de surveillance de conducteur comprenant :
une caméra configurée pour capturer des images de visage d'un conducteur dans un véhicule ; et
un processeur couplé à la caméra et configuré pour :
obtenir des séquences d'images à partir des images de visage, dans lequel chaque séquence d'images comporte une pluralité de scalaires de couleur associés à une pluralité de zones de visage du conducteur ;
déterminer, pour chaque séquence d'images, si une fonction de surveillance est activée pour un premier ensemble de zones de visage qui sont des zones de visage ombragées ; et
en réponse à la détermination que la fonction de surveillance est activée, réaliser les opérations suivantes :
la surveillance du premier ensemble de zones de visage ;
la détermination adaptative d'un second ensemble de zones de visage qui sont des zones de visage non ombragées, sur la base de résultats de la surveillance ; et
la réalisation de calculs de récupération de signaux de battements cardiaques uniquement pour le second ensemble de zones de visage.

11. Système de surveillance de conducteur selon la revendication 10, dans lequel le processeur est configuré pour calculer des premières valeurs SNR pour le premier ensemble de zones de visage.

12. Système de surveillance de conducteur selon la revendication 11, dans lequel le processeur est configuré pour :
comparer chaque première valeur SNR avec un seuil SNR ;
maintenir le second ensemble de zones de visage en réponse au fait que toutes les premières valeurs SNR sont inférieures au seuil SNR ; et
ajuster le second ensemble de zones de visage en réponse à une ou plusieurs premières valeurs SNR supérieures ou égales au seuil SNR.

13. Système de surveillance de conducteur selon la revendication 12, dans lequel le processeur est configuré pour :
déterminer une ou plusieurs zones de visage associées aux une ou plusieurs premières valeurs SNR supérieures ou égales au seuil SNR ; et
placer les une ou plusieurs zones de visage déterminées dans le second ensemble de zones de visage.

14. Système de surveillance de conducteur selon la revendication 10, dans lequel le processeur est configuré pour :
calculer des valeurs de zone pour le second ensemble de zones de visage ;
additionner les valeurs de zone calculées pour obtenir des valeurs finales ; et
récupérer les signaux de battements cardiaques sur la base des valeurs finales obtenues.

15. Support de stockage lisible par ordinateur comprenant des instructions exécutables par ordinateur qui, lorsqu'elles sont exécutées par un ordinateur, amènent l'ordinateur à réaliser le procédé selon l'une quelconque des revendications 1 à 9.
